# FASCICULE DE BREVET EUROPEEN

(11) **EP 3 215 130 B1**
(45) Date de publication et mention de la délivrance du brevet: **08.07.2020**
(21) Numéro de dépôt: 15807949.1
(22) Date de dépôt: 04.11.2015
(51) Int. Cl.: A61K 9/16, A61K 9/24, A61K 31/138, A61K 31/404, A61K 9/20, A61K 9/48

(54) **COMPOSITION PHARMACEUTIQUE COMPRENANT LE BISOPROLOL ET LE PÉRINDOPRIL**
PHARMAZEUTISCHE ZUSAMMENSETZUNG MIT BISOPROLOL UND PERINDORIL
PHARMACEUTICAL COMPOSITION COMPRISING BISOPROLOL AND PERINDORIL

(30) Priorité: 05.11.2014 FR 1460654
(43) Date de publication de la demande: 13.09.2017
(73) Titulaire: Les Laboratoires Servier, 92284 Suresnes (FR)
(72) Inventeur: FONKNECHTEN, Gilles, 45240 Ligny Le Ribault (FR)
(86) Numéro de dépôt international: PCT/FR2015/052975
(87) Numéro de publication internationale: WO 2016/071631

(56) Documents cités:
- WO-A2-2007/010501
- WO-A2-2010/038091

## Description

### Domaine technique

La présente invention concerne une composition pharmaceutique fixe comprenant un bétabloquant et un inhibiteur de l'enzyme de conversion de l'angiotensine (IEC) et, l'utilisation de ladite composition pour le traitement et la prévention de maladies cardiovasculaires et plus particulièrement l'hypertension artérielle, la maladie coronaire stable ou l'insuffisance cardiaque chronique.

Plus particulièrement, la présente invention concerne une composition pharmaceutique fixe comprenant un bétabloquant et un inhibiteur de l'enzyme de conversion de l'angiotensine (IEC) dans laquelle :
- le bétabloquant est le bisoprolol ou un de ses sels d'addition à un acide pharmaceutiquement acceptable, leurs hydrates et formes cristallines et,
- l'inhibiteur de l'enzyme de conversion est le périndopril ou un de ses sels d'addition à un acide ou à une base pharmaceutiquement acceptable, leurs hydrates et formes cristallines.

### Etat de la technique/contexte de l'invention

Les maladies cardiovasculaires sont la 1ère cause de mortalité aussi bien dans les pays développés qu'en développement, ils sont responsables d'un tiers des décès.

L'hypertension, la maladie coronaire et l'insuffisance cardiaque chronique sont des étapes majeures du continuum cardiovasculaire aboutissant au développement de maladies cardiaques de stade avancé. L'hypertension est la 1^{ère} cause de mortalité dans le monde causant environ 7,5 millions de décès (ce qui représente environ 13% des décès). La maladie coronaire est la conséquence la plus fréquente de l'hypertension et est la 1^{ère} cause de mortalité en Europe. L'insuffisance cardiaque est la pathologie terminale, conséquence de nombreuses maladies cardiaques. Le taux de mortalité reste actuellement encore élevé avec 50% de survie à 5 ans.

Par conséquent, une optimisation de la prise en charge et de la stratégie de traitement des patients est nécessaire.

Les maladies chroniques telles que les maladies cardiovasculaires requièrent souvent des polythérapies à long-terme. Ceci favorise la non-adhérence des patients à leur traitement, entrainant ainsi un mauvais contrôle de leurs pathologies.

Les associations fixes permettent une simplification du traitement en réduisant le nombre de comprimés quotidien et ont, par conséquent, le potentiel d'améliorer l'adhérence des patients au traitement.

La Société Européenne de Cardiologie (ESC = European Society of Cardiology) dans ses directives notamment celles de 2012 et 2013 recommande spécifiquement l'association de bétabloquants et d'IEC pour le traitement et/ou la prévention de l'hypertension artérielle chez les patients insuffisants cardiaques en post-infarctus ou souffrant de fibrillation auriculaire. Plus généralement, les bétabloquants et les IEC peuvent également être utilisés seuls ou en association en traitement d'initiation ou de maintenance de l'hypertension artérielle.

Pour le traitement de la maladie coronaire stable, 2 objectifs ont été exprimés à savoir :
a) Amélioration des symptômes et contrôle du rythme cardiaque : les bétabloquants sont indiqués en 1^{ère} intention (action directe sur le cœur, amélioration de la perfusion dans zone ischemiée) et
b) Prévention de l'apparition d'événements cardiovasculaires, en particuliers en cas de pathologie concomitante telle que l'hypertension, l'insuffisance cardiaque ou le diabète.

Pour le traitement de l'insuffisance cardiaque chronique (fraction d'éjection réduite) : les bétabloquants et les IEC sont complémentaires et sont recommandés chez tous les patients souffrant d'insuffisance cardiaque systolique. De plus, ils sont tous deux reconnus comme étant cardioprotecteurs chez les patients insuffisants cardiaques et en post-infarctus.

Le bisoprolol et le périndopril sont tous deux de principes actifs de médicaments approuvés et commercialisés depuis plusieurs années dans le monde. Leurs profils d'efficacité et de tolérance sont par conséquents bien établis. Les médicaments contenant ces deux principes actifs sont très utilisés dans la pratique médicale et très souvent co-prescrits. En effet, les médicaments en question ont des indications communes, à savoir l'hypertension, la maladie coronaire et l'insuffisance cardiaque.

De plus, leurs mécanismes d'action sont complémentaires et leurs effets bénéfiques sur la morbi-mortalité dans les maladies cardiovasculaires ont été démontrés dans des essais cliniques à grande échelle (l'étude CIBIS pour le bisoprolol et l'étude EUROPA pour le périndopril).

Parmi les bétabloquants qui sont co-prescrits avec le périndopril on trouve l'acebutolol, l'aténolol, le bisoprolol, le métoprolol et le nébivolol. Le bisoprolol est le bétabloquant qui est le plus co-prescrit avec le périndopril suivi par l'aténolol.

Les dosages de périndopril et de bisoprolol les plus co-prescrits dans les trois indications cibles de l'association, à savoir hypertension, maladie coronaire stable et insuffisance cardiaque chronique, sont indiqués dans le tableau ci-après :

**Tableau 1 : nombre de co-prescriptions de périndopril et de bisoprolol - IMS Health 2011-2013 ; 5 pays***

| | | **Prescriptions** | **% total** |
|---|---|---|---|
| **Total Perindopril + Bisoprolol** | | **1 894 791** | |
| **Périndopril 10 & 8 mg** | **Total Bisoprolol** | | |
| | **10 mg** | 161 675 | 8,5% |
| | 7,5 mg | 21 550 | 1,1% |
| | **5 mg** | 167 391 | 8,8% |
| | 3,75 mg | 13 616 | 0,7% |
| | 2,5 mg | 63 349 | 3,3% |
| | 1,25 mg | 44 623 | 2,4% |
| **Périndopril 5 & 4 mg** | **Total Bisoprolol** | | |
| | **10 mg** | 234 362 | 12,4% |
| | 7,5 mg | 13 819 | 0,7% |
| | **5 mg** | 502 932 | 26,5% |
| | 3,75 mg | 13 406 | 0,7% |
| | **2,5 mg** | 221 926 | 11,7% |
| | 1,25 mg | 145 617 | 7,7% |
| **Périndopril 2,5 & 2 mg** | **Total Bisoprolol** | 0 | |
| | 10 mg | 66 830 | 3,5% |
| | 7,5 mg | 7 233 | 0,4% |
| | 5 mg | 44 976 | 2,4% |
| | 3,75 mg | 19 126 | 1,0% |
| | 2,5 mg | 69 637 | 3,7% |
| | 1,25 mg | 82 723 | 4,4% |

| | | | |
|---|---|---|---|
| **IMS-Dec2011-Dec 2013: 5pays, France, Hongrie, Belgique, Italie, Roumanie Diag: I10, I11, I20.9, I25 & I50* | | | |

Les dosages de périndopril sont exprimés en sel d'arginine (2,5 mg, 5 mg ou 10 mg) ou en sel de *tert*-butylamine (2mg, 4 mg ou 8 mg) et les dosages de bisoprolol sont exprimés en base.

Le dosage de périndopril sel d'arginine 5 mg suivi du 10 mg est celui qui est le plus co-prescrit avec le bisoprolol 2,5 mg, 5 mg et 10mg.

Il y a donc un réel besoin d'une composition pharmaceutique fixe comprenant un bétabloquant et un IEC pour son utilisation dans le traitement et la prévention de maladies cardiovasculaires et plus particulièrement le traitement de l'hypertension artérielle, de la maladie coronaire stable et de l'insuffisance cardiaque chronique.

### Résumé de l'invention

La présente invention concerne une composition pharmaceutique fixe comprenant un bétabloquant et un inhibiteur de l'enzyme de conversion de l'angiotensine (IEC) et, l'utilisation de ladite composition pour le traitement et la prévention de maladies cardiovasculaires et plus particulièrement l'hypertension artérielle, la maladie coronaire stable ou l'insuffisance cardiaque chronique.

Le bétabloquant préférentiellement utilisé est le bisoprolol ou un de ses sels d'addition à un acide ou a une base pharmaceutiquement acceptable, et plus particulièrement son sel de fumarate, leurs hydrates et formes cristallines.

L'agent inhibiteur de l'enzyme de conversion de l'angiotensine préférentiellement utilisé est le périndopril ou un de ses sels d'addition à un acide ou à une base pharmaceutiquement acceptable, et plus particulièrement ses sels de *tert*-butylamine, tosylate ou d'arginine, leurs hydrates et formes cristallines.

### Description détaillée de l'invention

La présente invention concerne une composition pharmaceutique fixe comprenant le bisoprolol ou ses sels pharmaceutiquement acceptables, leurs hydrates et formes cristallines et le périndopril ou ses sels pharmaceutiquement acceptables, leurs hydrates et formes cristallines et, l'utilisation de ladite composition pour le traitement ou la prévention de maladies cardiovasculaires et plus particulièrement le traitement ou la prévention de l'hypertension artérielle, de la maladie coronaire stable ou de l'insuffisance cardiaque chronique.

Le bisoprolol ou (R,S)1-[4-[[2-(1-méthyléthoxy)éthoxy]méthyl]phénoxy]-3-[1-méthyléthyl)amino]-2-propanol, de formule (I) : 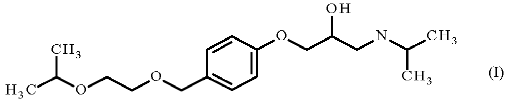 est un bétabloquant. Le bisoprolol a été précédemment décrit dans le brevet français FR 2 367 053 ou US 4 258 062. Le bisoprolol fumarate est une poudre blanche de formule (la) :

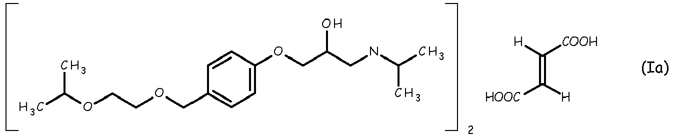

Le bisoprolol fumarate qui est commercialisé sous le nom de Cardensiel® est indiqué dans le traitement de l'insuffisance cardiaque chronique stable avec réduction de la fonction ventriculaire systolique gauche, en complément des inhibiteurs de l'enzyme de conversion (IEC) et des diurétiques et, éventuellement, des digitaliques. Le Cardensiel® est un comprimé pelliculé sécable ou non obtenu par compression directe conventionnelle. Les différents dosages disponibles du Cardensiel® sont 1,25 mg, 2,5 mg, 3,75 mg, 5 mg, 7,5 mg et 10 mg exprimés en bisoprolol fumarate avec une posologie initiale de 1,25 mg une fois par jour puis on augmente progressivement jusqu'à 10mg qui est la dose de maintenance. Quelle que soit la dose, la posologie est toujours de une prise par jour.

Deux associations fixes contenant du bisoprolol sont actuellement commercialisées : Lodoz® et Asabis® :
(a) L'association du bisoprolol fumarate et de l'hydrochlorothiazide qui est commercialisée sous le nom de Lodoz® est indiquée pour l'hypertension artérielle légère à modérée. Le Lodoz® est un comprimé pelliculé en monocouche obtenu par compression directe conventionnelle. Les différents dosages disponibles du Lodoz® sont :

| Lodoz® | Par comprimé (en mg) | | |
|---|---|---|---|
| Bisoprolol fumarate | 2,5 | 5 | 10 |
| Hydrochlorothiazide | 6,25 | 6,25 | 6,25 |

(b) L'association du bisoprolol fumarate et de l'acide acétylsalicylique qui est commercialisé sous le nom de Asabis® est indiquée pour le traitement de l'hypertension chez les patients préalablement stabilisés avec les composants individuels et, le traitement de l'angine de poitrine chez les patients préalablement stabilisés avec les composants individuels. Asabis® est une gélule contenant du bisoprolol fumarate qui est soit sous forme de simple mélange bisoprolol/excipients soit sous forme de granules et de l'acide acétylsalicylique sous forme de comprimé pelliculé. Les différents dosages disponibles de Asabis® sont :

| Asabis® | Par comprimé (en mg) | |
|---|---|---|
| Bisoprolol fumarate | 5 | 10 |
| Acide acétylsalicylique | 75 | 75 |

La demande de brevet EP 2 359 815 décrit l'association de bisoprolol fumarate et d'amlodipine besilate ainsi que son utilisation pour le traitement de l'hypertension et de l'angine de poitrine. La composition pharmaceutique comprenant le bisoprolol et l'amlodipine est soit un comprimé monocouche obtenu par compression directe conventionnelle soit, une gélule dans laquelle le bisoprolol et l'amlodipine sont sous forme de poudre.

Le périndopril ou acide (2S)-2-[(1S)-carbéthoxybutylamino]-1-oxopropyl (23,3aS,7aS)perhydroindole carboxylique, de formule (II) : 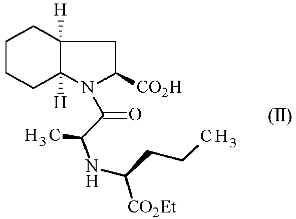 est un inhibiteur de l'enzyme de conversion de l'angiotensine I (IEC). Le périndopril a été précédemment décrit dans le brevet EP 0 049 658. Dans ce brevet européen, de manière classique, il est mentionné que les composés de l'invention peuvent se présenter sous forme de sels d'addition avec une base ou un acide minéral ou organique pharmaceutiquement acceptable. Les brevets EP 0 308 341, EP 1 256 590, EP 1 268 424, EP 1 279 665, EP 1 321 471, EP 1 333 026, EP 1 362 864, EP 1 367 061, EP 1 367 062, EP 1 367 063, EP 1 371 659, EP 1 380 590, EP 1 380 591,EP 1 403 275, EP 1 420 028, EP 1 420 029, EP 1 422 236, EP 1 603 558 et EP 1 7533 720 décrivent une méthode d'obtention de sels de périndopril, plus spécifiquement le sel de tert-butylamine. Le sel de L-arginine du périndopril de formule (IIa) a été décrit pour la première fois dans le brevet européen EP 1 354 873 :

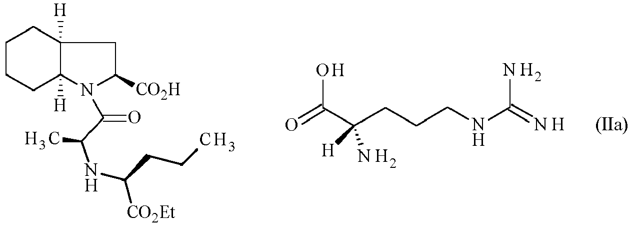

Les formes cristallines alpha et bêta du sel de L-arginine du périndopril ont été décrites dans les brevets européens EP 1 989 182 et EP 2 016 051 respectivement, la forme cristalline delta a été décrite dans le brevet européen EP 2 612 850 et la forme cristalline gamma a été décrite dans la demande internationale WO 2009/157018.

Le périndopril qui est commercialisé sous le nom de Coversyl® est indiqué dans le traitement de l'hypertension artérielle, de la maladie coronaire stable notamment pour le risque d'événements cardiaques chez les patients ayant un antécédent d'infarctus du myocarde et/ou revascularisation et, de l'insuffisance cardiaque notamment pour le traitement de l'insuffisance cardiaque symptomatique. Le Coversyl® est un comprimé monocouche pelliculé sécable ou non sécable. Les différents dosages disponibles du Coversyl® sont: 2,5 mg, 5 mg, et 10 mg exprimés en périndopril arginine ou encore 1,6975 mg/cp 2,5 mg; 3,395 mg/cp 5 mg ; 6,790 mg/cp 10 mg exprimé en périndopril. La posologie doit être adaptée au patient. Cependant, la dose initiale recommandée est de 5 mg par jour en une prise matinale.

Deux associations fixes contenant du périndopril sont actuellement commercialisées : Coveram® et Bipreterax® :
(a) L'association du périndopril arginine et de l'amlodipine besilate qui est commercialisée sous le nom de Coveram® est indiquée pour le traitement de l'hypertension artérielle essentielle et/ou de la maladie coronaire stable, en substitution, chez les patients déjà contrôlés avec le périndopril et l'amlodipine pris simultanément à la même posologie. Le Coveram® est un comprimé monocouche. Les différents dosages disponibles du Coveram® sont :

| Coveram® | Par comprimé (en mg) | | | |
|---|---|---|---|---|
| Périndopril arginine | 5 | 5 | 10 | 10 |
| (soit en périndopril : 3,395 mg/cp à 5 mg de périndopril arginine ou 6,790 mg/cp à 10 mg de périndopril arginine) | | | | |
| Amlodipine | 5 | 10 | 5 | 10 |
| (sous forme de bésilate d'amlodipine : 6,935 mg/cp à 5 mg d'amlodipine ou 13,870 mg/cp à 10 mg d'amlodipine) | | | | |

(b) L'association du périndopril arginine et de l'indapamide qui est commercialisée sous le nom de Bipreterax® est indiquée pour le traitement de l'hypertension artérielle essentielle et est aussi indiqué chez les patients pour lesquels la pression artérielle est insuffisamment contrôlée par le périndopril seul. Le Bipreterax® est un comprimé pelliculé monocouche. Les différents dosages disponibles du Bipreterax® sont :

| Bipreterax® | Par comprimé (en mg) | |
|---|---|---|
| Périndopril arginine | 5 | 10 |
| (soit en périndopril : 3,395 mg/cp à 5 mg de périndopril arginine ou 6,790 mg/cp à 10 mg de périndopril arginine) | | |
| Indapamide | 1,25 | 5 |

Une autre association fixe avec le périndopril qui vient d'obtenir une autorisation de mise sur le marché (AMM) en Europe est le Triplixam®.

L'association du périndopril arginine, d'inpadamide et de l'amlodipine besilate qui est commercialisé sous le nom de Triplixam® est indiquée pour le traitement de l'hypertension artérielle essentielle, en substitution, chez les patients déjà contrôlée avec l'association à dose fixe périndopril/indapamide et l'amlodipine, pris simultanément aux mêmes posologies. Le Triplixam® est un comprimé monocouche. Les différents dosages disponibles du Triplixam® sont :

| Triplixam® | Par comprimé (en mg) | | | | |
|---|---|---|---|---|---|
| Périndopril arginine | 2,5 | 5 | 5 | 10 | 10 |
| (soit en périndopril : 1,6975 mg/cp à 2,5 mg de périndopril arginine ou 3,395 mg/cp à 5 mg de périndopril arginine ou 6,790 mg/cp à 10 mg de périndopril arginine) | | | | | |
| Indapamide | 0,6258 | 1,25 | 1,25 | 2,5 | 2,5 |
| Amlodipine | 5 | 5 | 10 | 5 | 10 |
| (sous forme de bésilate d'amlodipine : 6,935 mg/cp à 5 mg d'amlodipine ou 13,870 mg/cp à 10 mg d'amlodipine) | | | | | |

Le brevet EP 1 800 678 décrit l'association de l'ivabradine et du périndopril ainsi que son utilisation pour le traitement de l'hypertension artérielle. La demande de brevet EP 2 404 600 décrit l'utilisation de cette même association pour le traitement de l'insuffisance cardiaque.

WO 2007/010501 divulgue des compositions pharmaceutiques fixes sous forme de gélules combinant le métoprolol et le ramipril, le métoprolol étant présent sous forme de libération prolongée et le ramipril étant présent sous forme de libération immédiate. Il divulgue le bisoprolol et le périndopril parmi les bétabloquants et les inhibiteurs d'IEC, respectivement pouvant être combinés. Il divulgue des comprimés et des gélules où les deux principes actifs ne sont pas en contact directe. Cependant le but de la séparation n'est pas de limiter le contact entre les deux principes actifs ou d'éviter des problèmes de sous-dosage mais d'obtenir une libération appropriée de chaque principe actif, c'est-à-dire prolongée dans le cas du métoprolol et immédiate dans le cas du ramipril. WO 2007/010501 n'adresse pas de problèmes d'interaction entre les deux principes actifs et divulgue en général des compositions sous forme de comprimés pelliculés obtenus par compression directe, des comprimés bicouches et des compositions sous forme de gélules. C'est le seul document qui divulgue une telle composition fixe mais, il ne divulgue pas ni ne suggère la combinaison spécifique de bisoprolol et périndopril.

C'est donc un réel besoin pour les patients souffrants de maladies cardiovasculaires telles que l'hypertension artérielle, la maladie coronaire stable ou encore l'insuffisance cardiaque chronique de bénéficier d'une telle composition pharmaceutique fixe.

Une composition pharmaceutique fixe a l'avantage de permettre à la fois une réduction des coûts de fabrication, mais surtout une meilleure observance du traitement de la part des patients et par conséquent un meilleur contrôle de leur pathologie.

Cependant, un des risques majeurs des combinaisons pharmaceutiques fixes résulte d'une possible interaction entre les différents principes actifs présents dans cette combinaison.
En dehors des excipients, lorsque les principes actifs sont formulés seuls, bisoprolol seul ou périndopril seul, ils peuvent contenir des impuretés et/ou des produits de dégradation dans les produits de départ mais aussi des impuretés potentielles dans le produit final. Par exemple, une des impuretés organiques qui résulte de la voie de synthèse du bisoprolol est l'epoxibiso ou 1-[4-[(2-isopropoxyethoxy)methyl]phenoxy]-2,3-epoxypropane de formule (Ib) :

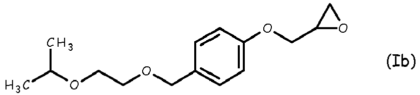

Cette impureté epoxibiso est toujours présente dans le produit de départ.

Lorsque les deux principes actifs sont présents dans une composition pharmaceutique fixe, en plus des contraintes liées aux principes actifs seuls, il peut y avoir des problèmes de sous-dosages dans la composition finale mais aussi des problèmes de stabilité de ces principes actifs ainsi que l'apparition de nouvelles impuretés en plus des impuretés déjà connues des principes actifs pris isolément.

Toutes les associations fixes de périndopril actuellement sur le marché et Triplixam® qui vient d'obtenir son AMM sont des comprimés monocouches. Aucun problème de sous-dosage des principes actifs ou encore de stabilité ou de formation de nouvelles impuretés des compositions pharmaceutiques fixes n'a été reporté.

La demanderesse a donc tout d'abord envisagé une composition pharmaceutique fixe de bisoprolol et de périndopril sous forme de comprimé monocouche.

Dans un premier temps, pour réaliser l'invention la demanderesse a envisagé une composition pharmaceutique fixe de bisoprolol et de périndopril sous forme de comprimé monocouche obtenu par compression directe (exemple A) ou par granulation humide (exemple B). De façon surprenante, la demanderesse a pu constater que les formulations testées n'ont pas donné lieu à des résultats satisfaisants d'un point de vue du dosage (tableau 2) et/ou de la stabilité (tableau 3).

**Tableau 2 : Teneur en principes actifs dans les comprimés monocouches obtenus par compression directe**

| **Etape de compression / niveau de prélèvement** | **Teneur en bisoprolol fumarate (en %)** | **Teneur en périndopril arginine (en %)** | **Spécifications (en %)** |
|---|---|---|---|
| Début compression | 102,40 | 109,20 | 95 - 105 |
| Milieu compression | 97,60 | 97,20 | |
| Fin compression | 95,20 | 92,80 | |

Ces résultats ont été obtenus à partir des comprimés non pelliculés dosés à 1,25 mg de bisoprolol et 2,5 mg de périndopril sel d'arginine. Les comprimés ont été prélevés directement à la sortie de la presse à comprimés et ce à différents moments de la compression.

L'analyse de la teneur en principe actif a été effectuée par dosage des deux principes actifs par chromatographie liquide avec détection UV, soit par chromatographie en phase liquide à haute performance (HPLC) sur colonne Inertsil® C8 (longueur 15 cm, diamètre : 4,6 mm; porosité 150A; taille de particules 5 micromètres), soit par chromatographie en phase liquide à ultra performance (UPLC) sur colonne Kinetec® C8 (longueur 10 cm, diamètre : 3,0 mm; porosité 100A; taille de particules 2,6 micromètres).

Les analyses des comprimés monocouches ont révélé une non-conformité en début et en fin de compression avec une chute importante de la teneur en principes actifs en fin de compression (tableau 2). L'analyse des comprimés monocouches obtenus par granulation humide s'est révélée conforme aux spécifications à savoir un dosage en principes actifs compris entre 95 et 105 % de la dose théorique.

Puisque les comprimés monocouches obtenus par compression directe n'ont pas été conformes aux spécifications alors que les comprimés monocouches obtenus par granulation humide étaient quant à eux conformes quant à la teneur en principes actifs, la demanderesse a étudié uniquement la stabilité des comprimés monocouches obtenus par granulation humide (tableau 3).

Les analyses effectuées ont pu mettre en évidence la formation de nombreux produits de dégradation connus et inconnus au cours du temps lorsque les comprimés monocouches sont obtenus par granulation humide (tableau 3).

**Tableau 3 : Stabilité des comprimés monocouches obtenus par granulation humide après 6 mois à 40°C/75% HR**

| | **Quantité en %** | | |
|---|---|---|---|
| **Dosage** | **Cp x mg bisoprolol fumarate / y mg périndopril arginine** | | |
| | **Cp 10/10 mg (T0)** | **Cp 10/10 mg (T6)** | **Spécifications (en %)** |
| Impuretés du bisoprolol fumarate (en %) | | | |
| Total impuretés* | 0,2 | 4,7 | ≤ 6,00 |

| Impuretés du périndopril arginine (en %) | | | |
|---|---|---|---|
| Total impuretés* | 0,5 | 5,20 | ≤ 5,00 |

| | | | |
|---|---|---|---|
| * impuretés = produits de dégradation + impuretés inconnues | | | |

Ces résultats ont été obtenus à partir de comprimés pelliculés conditionnés en piluliers de 30 comprimés placés ensuite en étuves dont la température et le taux d'humidités sont contrôlés.

Les comprimés monocouches obtenus par granulation humide sont correctement dosés en actifs. En ce qui concerne les teneurs en impuretés du bisoprolol, la quantité totale d'impuretés a été multipliée par plus de 20 entre T0 et T6 mois avec émergence de 9 impuretés inconnues dont 6 hors-spécification.

Pour le périndopril, bien qu'aucune impureté inconnue ne soit présente, la quantité totale d'impuretés a été multipliée par 10 entre T0 et T6 mois et a dépassé la limite de spécification.

En somme, ces résultats mettent en évidence l'instabilité dans le temps de la composition pharmaceutique fixe, le comprimé monocouche obtenu par granulation humide.

De façon tout à fait surprenante, la demanderesse s'est rendue compte qu'une séparation physique du bisoprolol et du périndopril dans une composition pharmaceutique fixe permettait de proposer une solution aux inconvénients précédemment mentionnés.

Il existe différentes façons de réaliser une séparation physique de deux principes actifs ou plus dans une composition pharmaceutique fixe.

On peut soit réaliser une composition pharmaceutique fixe qui est un comprimé bicouche où chacune des couches contient un seul des deux principes actifs. Une première couche contient le bisoprolol seul et une deuxième couche contient le périndopril seul. Cette composition pharmaceutique fixe limite le contact entre le bisoprolol et le périndopril à la seule interface entre les deux couches. Bien qu'il existe potentiellement une interaction entre les deux couches, celle-ci est très faible et ne doit pas conduire à des taux de dégradation importants.

Soit, on peut réaliser une composition pharmaceutique fixe qui est un comprimé tricouche comprenant en plus des deux couches distinctes une couche de séparation se trouvant entre la couche de bisoprolol et la couche de périndopril.

Une autre solution consiste à réaliser une composition pharmaceutique fixe qui est une gélule dans laquelle les deux principes actifs sont sous formes de granules de bisoprolol et granules de périndopril, de comprimés de bisoprolol et comprimés de périndopril ou un mélange des deux à savoir des comprimés de l'un des principes actifs et des granules de l'autre des principes actifs.

Un aspect de l'invention est donc une composition pharmaceutique fixe comprenant du bisoprolol et ses sels pharmaceutiquement acceptables et, du périndopril et ses sels pharmaceutiquement acceptables seuls ou en combinaison avec un ou plusieurs excipients pharmaceutiquement acceptables, leurs hydrates et leurs formes cristallines.

Selon un autre aspect de l'invention la composition pharmaceutique fixe est un comprimé bicouche, un comprimé tricouche ou une gélule.

De manière préférentielle, la composition pharmaceutique fixe est un comprimé bicouche où chacune des couches contient un des deux principes actifs.

Encore un autre aspect de l'invention est une composition pharmaceutique fixe qui est un comprimé tricouche qui est le comprimé bicouche qui comprend en outre une couche de séparation entre les deux couches qui contiennent les deux principes actifs.

Encore un autre aspect de l'invention est une composition pharmaceutique fixe qui est une gélule qui contient des granules ou des comprimés ou un mélange de granules et de gélules de chacun des deux principes actifs.

Un autre aspect de l'invention est une composition pharmaceutique fixe dans laquelle le bisoprolol est préférentiellement sous forme de bisoprolol fumarate.

Encore un autre aspect de l'invention est une composition pharmaceutique fixe dans laquelle le périndopril est préférentiellement sous forme de périndopril *tert*-butylamine ou d'arginine et encore plus préférentiellement sous forme de périndopril arginine.

La présente invention s'étend également aux compositions pharmaceutiques fixes comprenant du bisoprolol et ses sels pharmaceutiquement acceptables et, du périndopril et ses sels pharmaceutiquement acceptables seuls ou en combinaison avec un ou plusieurs excipients pharmaceutiquement acceptables, leurs hydrates et leurs formes cristallines dans lesquelles :
- les doses de bisoprolol sont de 1,05 mg et 8,50 mg exprimées en bisoprolol base ou entre 1,25 et 10 mg exprimées en bisoprolol fumarate et,
- les doses de périndopril sont de 1,65 et 6,80 mg exprimées en périndopril base ou entre 2,5 à 10 mg exprimées en périndopril arginine.

De manière préférentielle, dans les compositions pharmaceutiques fixes, les doses de bisoprolol fumarate sont de 2,5 mg, 5 mg et 10 mg et les doses de périndopril arginine sont de 2,5 mg, 5 mg et 10 mg.

De manière encore plus préférentielle, dans les compositions pharmaceutiques fixes, les doses sont les suivantes :
a) 2,5 mg de bisoprolol fumarate et 2,5 mg de périndopril arginine
b) 2,5 mg de bisoprolol fumarate et 5 mg de périndopril arginine
c) 5 mg de bisoprolol fumarate et 5 mg de périndopril arginine
d) 5 mg de bisoprolol fumarate et 10 mg de périndopril arginine
e) 10 mg de bisoprolol fumarate et 5 mg de périndopril arginine
f) 10 mg de bisoprolol fumarate et 10 mg de périndopril arginine

La présente invention s'étend également aux compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines pour leur utilisation dans le traitement ou la prévention de maladie cardiovasculaires.

De manière préférentielle, la présente invention s'étend également aux compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines pour leur utilisation dans le traitement de maladie cardiovasculaires.

De manière préférentielle, la présente invention s'étend également aux compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines pour leur utilisation dans la prévention de maladie cardiovasculaires.

Encore un autre aspect de l'invention est l'utilisation des compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines dans le traitement ou la prévention de l'hypertension artérielle, la maladie coronaire stable ou l'insuffisance cardiaque chronique.

Encore un autre aspect de l'invention est l'utilisation des compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines dans le traitement ou la prévention de l'hypertension artérielle.

Encore un autre aspect de l'invention est l'utilisation des compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines dans le traitement ou la prévention de la maladie coronaire stable.

Encore un autre aspect de l'invention est l'utilisation des compositions pharmaceutiques fixes comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines dans le traitement ou la prévention de l'insuffisance cardiaque chronique.

Outre le bétabloquant et le composé inhibiteur de l'enzyme de conversion de l'angiotensine (l'IEC), lesdites compositions pharmaceutiques contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la cellulose, la méthylcellulose, l'hydroxypropylcellulose, l'hydroxypropylmethylcellulose, et la polyvinylpyrrolidone,
◆ *pour les désintégrants* : l'agar, l'amidon, l'acide alginique et son sel de sodium, les mélanges effervescents, les sels de carboxyméthylcellulose, les sels de carboxyméthylamidon, les dérivés de la polyvinylpyrrolidone.

La posologie utile varie selon l'âge du patient, la nature de l'affection et, les pathologies et traitements éventuellement associés.

Pour l'hypertension et la maladie coronaire stable, la posologie usuelle du bisoprolol est de 5 mg/jour. La dose peut être augmentée à 10 mg/jour si nécessaire. La dose maximale recommandée est de 20 mg/jour. Dans l'insuffisance cardiaque chronique, une phase de titration des doses est nécessaire avec une dose initiale à 1,25 mg/jour augmentée progressivement en fonction de la tolérance du patient pour atteindre la dose de maintenance de 10 mg/jour. La dose maximale recommandée est de 10 mg/jour.

La posologie usuelle du périndopril sel d'arginine s'échelonne de 2,5 mg à 10 mg/jour (2 mg à 8 mg/jour pour le périndopril sel de *tert*-butylamine) avec une posologie initiale recommandée de 5 mg/jour qui peut être augmentée à 10 mg/jour pour l'hypertension artérielle et la maladie coronaire stable et une posologie initiale de 2,5 mg/jour qui peut être augmentée à 5 mg/jour dans l'insuffisance cardiaque.

Les exemples suivants illustrent l'invention et ne la limitent en aucune façon.

### Exemples

Les exemples A et B sont des comparateurs
**Exemples A :** comprimé monocouche dosé à 10/10 mg obtenu par compression directe

| **Formule** | **En %** | **En mg par comprimé** |
|---|---|---|
| Bisoprolol fumarate | 4,17 | 10,00 |
| Perindopril arginine | 4,17 | 10,00 |
| Carbonate de calcium/amidon | 56,63 | 131,11 |
| Cellulose microcristalline (PH 102) | 33,33 | 80,00 |
| Carboxyméthylamidon sodique | 3,00 | 7,20 |
| Silice colloïdale anhydre | 0,20 | 0,48 |
| Stéarate de magnésium | 0,50 | 1,20 |
| **Total** | **100** | **240** |

**Exemple B** : comprimé monocouche dosé à 10/10 mg obtenu par granulation humide.

| **Formule** | **En %** | **En mg par comprimé** |
|---|---|---|
| Bisoprolol fumarate | 4,17 | 10,00 |
| Perindopril arginine | 4,17 | 10,00 |
| Cellulose microcristalline | 15,00 | 36,00 |
| Carbonate de calcium | 29,63 | 71,12 |
| Eau purifiée | qs | Qs |
| Cellulose microcristalline (PH 102) | 33,33 | 80,00 |
| Carboxyméthylamidon sodique | 3,00 | 7,20 |
| Silice colloïdale anhydre | 0,20 | 0,48 |
| Stéarate de magnésium | 0,50 | 1,20 |
| **Total** | **100** | **240** |

Les exemples qui suivent font partie de l'invention.

### Exemple 1 : formule des quatre dosages des comprimés bicouches

**Exemple 1a** : comprimés bicouches dosés à 5 mg de bisoprolol et 5 mg de périndopril
**Exemple 1b** : comprimés bicouches dosés à 5 mg de bisoprolol et 10 mg de périndopril
**Exemple 1c** : comprimés bicouches dosés à 10 mg de bisoprolol et 5 mg de périndopril
**Exemple 1d** : comprimés bicouches dosés à 10 mg de bisoprolol et 10 mg de périndopril

| | **Quantité en %** | | | |
|---|---|---|---|---|
| **Dosage** | **Cp x mg bisoprolol fumarate / y mg périndopril arginine** | | | |
| | **Cp 5/5 mg** | **Cp 5/10 mg** | **Cp 10/5 mg** | **Cp 10/10 mg** |
| **Couche bisoprolol fumarate** | | | | |
| Bisoprolol fumarate | 4,03 | 2,68 | 5,37 | 4,03 |
| Cellulose microcristalline | 16,11 | 10,74 | 21,48 | 16,11 |
| Carbonate de calcium | 18,45 | 12,30 | 24,60 | 18,45 |
| Amidon de maïs | 7,73 | 5,15 | 10,31 | 7,73 |
| Carboxyméthylamidon sodique | 1,45 | 0,97 | 1,93 | 1,45 |
| Silice colloïdale anhydre | 0,19 | 0,13 | 0,26 | 0,19 |
| Stéarate de magnésium | 0,36 | 0,24 | 0,48 | 0,36 |

| **Couche périndopril arginine** | | | | |
|---|---|---|---|---|
| Périndopril arginine | 4,03 | 5,37 | 2,68 | 4,03 |
| Cellulose microcristalline | 16,11 | 21,48 | 10,74 | 16,11 |
| Carbonate de calcium | 18,45 | 24,60 | 12,30 | 18,45 |
| Amidon de maïs prégélatinisé | 7,73 | 10,31 | 5,15 | 7,73 |
| Sodium croscarmellose | 1,45 | 1,93 | 0,97 | 1,45 |
| Silice colloïdale anhydre | 0,19 | 0,26 | 0,13 | 0,19 |
| Stéarate de magnésium | 0,36 | 0,48 | 0,24 | 0,36 |

| **Comprimé pelliculé** | | | | |
|---|---|---|---|---|
| Premix pour enrobage brun 5943 | 3,25 | 3,25 | 3,25 | 3,25 |
| Macrogol 6000 | 0,10 | 0,10 | 0,10 | 0,10 |

### Procédé de pelliculage et de lissage

Le procédé de pelliculage et de lissage du comprimé nu consiste principalement en 2 étapes qui sont: a) l'étape de pelliculage puis b) l'étape de lissage.

Les techniques utilisées sont des techniques conventionnelles de pelliculage et de lissage bien connues de l'homme de l'art.

### Exemple 2 : Comprimé pelliculé sécable

Parmi les compositions pharmaceutiques fixes décrites dans l'exemple 1, deux d'entre elles (les exemples la et 1b) comportent une barre de sécabilité ou barre de cassure. Les compositions pharmaceutiques fixes en question sont celles comprenant :
a) L'exemple 1a : 5 mg de bisoprolol fumarate et 5 mg de périndopril arginine et,
b) L'exemple 1b : 5 mg de bisoprolol fumarate et 10 mg de périndopril arginine.

| **Cp x mg de bisoprolol/y mg de périndopril** | | | |
|---|---|---|---|
| **Cp 5/5** | **Cp 5/10** | **Cp 10/5** | **Cp 10/10** |
| pelliculé brun | | | |
| Elliptique Sécable | Elliptique Sécable | Rond | Elliptique |

L'avantage de cette barre de sécabilité, est la possibilité de mettre à la disposition des patients les dosages suivants :
a) 2,5 mg de bisoprolol fumarate et 2,5 mg de périndopril arginine et,
b) 2,5mg de bisoprolol fumarate et 5 mg de périndopril arginine.

Les compositions pharmaceutiques fixes de bisoprolol et de périndopril selon l'invention (les exemples 1 et 2) permettent ainsi de mettre à disposition des patients au moins 71% des co-prescriptions des composés pris individuellement.

### Résultats de stabilité à 6 mois des comprimés bicouches

Les résultats de stabilité des comprimés bicouches à 6 mois ont été obtenus à partir des comprimés pelliculés conditionnés en piluliers de 30 comprimés placés ensuite en étuves dont la température et le taux d'humidité (HR) ont été contrôlés. Ces paramètres sont de 40°C pour la température et de 75 % pour le taux d'humidité.

La séparation des deux principes actifs a permis de corriger les problèmes de sous-dosage en principes actifs rencontrés avec le comprimé monocouche et surtout d'obtenir une forme pharmaceutique stable dans le temps (tableau 5).

**Tableau 5 : comparaison des résultats de stabilités à 6 mois - 40°C/75% HR des comprimés monocouches et comprimés bicouche à la dose de 10/10 mg**

| | **Quantité en %** | | |
|---|---|---|---|
| **Dosage** | **Comprimé monocouche** (exemple B) | **Comprimé bicouche** (exemple 1d) | **Spécifications (en %)** |
| Impuretés* du bisoprolol fumarate (en %) | | | |
| Total impuretés | 4,70 | 0,20 | ≤ 6,00 |

| Impuretés* du périndopril arginine (en %) | | | |
|---|---|---|---|
| Total impuretés | 5,20 | 0,30 | ≤ 5,00 |

| | | | |
|---|---|---|---|
| * impuretés = produits de dégradation + impuretés inconnues | | | |

En effet, les études de stabilité ont montré une diminution importante de la quantité totale d'impuretés de dégradation pour le bisoprolol fumarate et le périndopril arginine ainsi que, pour le bisoprolol fumarate, une nette réduction du nombre d'impuretés inconnues.

La quantité totale d'impuretés pour un comprimé dosé à 10/10 mg est passée de 4,70 % dans le comprimé monocouche à 0,20 % dans le comprimé bicouche pour le bisoprolol et de 5,20% dans le comprimé monocouche à 0,30 % dans le comprimé bicouche pour le périndopril.

### Exemple 3 : Comprimé tricouche avec une couche de séparation

Le comprimé tricouche a été réalisé de la même manière que le comprimé bicouche en prenant les comprimés des exemples la à 1d et en rajoutant en outre une couche de séparation entre la couche de bisoprolol et la couche de périndopril. Cette couche de séparation contient un mélange de lactose et de stéarate de magnésium.

### Exemple 4 : Gélules de bisoprolol et de périndopril

La composition pharmaceutique pour une gélule qui contient 10 mg de bisoprolol fumarate sous forme de granules et 10 mg de périndopril sel d'arginine sous forme de granules est la suivante :
a) Granules de 10 mg de bisoprolol fumarate

| | |
|---|---|
| Bisoprolol fumarate ......................... | 10 mg |
| Hydroxypropylméthylcellulose ........ | 1,1 mg |
| Sphères de sucre .............................. | 88,89 mg |

b) Granules de 10 mg de périndopril sel d'arginine

| | |
|---|---|
| Périndopril sel d'arginine................. | 10 mg |
| Hydroxypropylcellulose................... | 4,25 mg |
| Sphères de sucre ........................... | 45,70 mg. |

## Revendications

1. Composition pharmaceutique fixe comprenant du bisoprolol et ses sels pharmaceutiquement acceptables et, du périndopril et ses sels pharmaceutiquement acceptables en combinaison avec un ou plusieurs excipients pharmaceutiquement acceptables, leurs hydrates et leurs formes cristallines dans laquelle le bisoprolol et le périndopril sont séparés physiquement.

2. Composition pharmaceutique fixe selon la revendication 1 qui est un comprimé bicouche dans lequel une première couche contient le bisoprolol et une deuxième couche contient le périndopril, un comprimé tricouche dans lequel en plus des deux couches distinctes on a une couche de séparation, ou une gélule comprenant des granules de bisoprolol et des granules de périndopril, des comprimés de bisoprolol et des comprimés de périndopril ou des comprimés de l'un des principes actifs et des granules de l'autre des principes actifs.

3. Composition pharmaceutique fixe selon l'une des revendications 1 ou 2 dans laquelle la composition pharmaceutique est un comprimé bicouche dans lequel une première couche contient le bisoprolol et une deuxième couche contient le périndopril.

4. Composition pharmaceutique fixe selon l'une des revendications 1 ou 2 dans laquelle la composition pharmaceutique est un comprimé tricouche dans lequel en plus des deux couches distinctes on a une couche de séparation.

5. Composition pharmaceutique fixe selon l'une des revendications 1 ou 2 dans laquelle la composition pharmaceutique est une gélule comprenant des granules de bisoprolol et des granules de périndopril, des comprimés de bisoprolol et des comprimés de périndopril ou des comprimés de l'un des principes actifs et des granules de l'autre des principes actifs.

6. Composition pharmaceutique fixe selon l'une des revendications 1 à 5 dans laquelle le bisoprolol et sous forme de bisoprolol fumarate.

7. Composition pharmaceutique fixe selon l'une des revendications 1 ou 5 dans laquelle le périndopril est sous forme de périndopril *tert*-butylamine ou périndopril arginine et plus préférentiellement sous forme de périndopril arginine.

8. Composition pharmaceutique fixe selon l'une des revendications 1 à 7 dans lesquelles :
- les doses de bisoprolol sont de 1,05 mg et 8,50 mg exprimées en bisoprolol base ou entre 1,25 et 10 mg exprimées en bisoprolol fumarate et,
- les doses de périndopril sont de 1,65 et 6,80 mg exprimées en périndopril base ou entre 2,5 à 10 mg exprimées en périndopril arginine.

9. Composition pharmaceutique fixe selon l'une des revendications 1 à 8 dans laquelle les doses de bisoprolol fumarate sont de 2,5 mg, 5 mg et 10 mg et les doses de périndopril arginine sont de 2,5 mg, 5 mg et 10 mg.

10. Compositions pharmaceutiques fixes selon l'une des revendication 1 à 9 pour leur utilisation dans le traitement ou la prévention de maladies cardiovasculaires.

11. Compositions pharmaceutiques fixes selon la revendication 10 **caractérisées en ce que** les maladies cardiovasculaires sont choisies parmi l'hypertension artérielle, la maladie coronaire stable ou l'insuffisance cardiaque chronique.

## Patentansprüche

1. Pharmazeutische Festdosis-Zubereitung, umfassend Bisoprolol und seine pharmazeutisch annehmbaren Salze, und Perindopril und seine pharmazeutisch annehmbaren Salze in Kombination mit einem oder mehreren pharmazeutisch annehmbaren Hilfsstoffen, ihren Hydraten und ihren Kristallformen, wobei Bisoprolol und Perindopril physikalisch getrennt sind.

2. Pharmazeutische Festdosis-Zubereitung nach Anspruch 1, welche eine Zweischicht-Tablette ist, bei der eine erste Schicht Bisoprolol enthält und eine zweite Schicht Perindopril enthält, eine Dreischicht-Tablette ist, bei der zusätzlich zu den beiden getrennten Schichten eine Trennschicht vorliegt, oder eine Gelkapsel ist umfassend Bisoprolol-Granulat und Perindopril-Granulat, Bisoprolol-Tabletten und Perindopril-Tabletten oder Tabletten eines der Wirkstoffe und ein Granulat des anderen Wirkstoffs.

3. Pharmazeutische Festdosis-Zubereitung nach einem der Ansprüche 1 oder 2, wobei die pharmazeutische Zubereitung eine Zweischicht-Tablette ist, bei der eine erste Schicht Bisoprolol enthält und eine zweite Schicht Perindopril enthält.

4. Pharmazeutische Festdosis-Zubereitung nach einem der Ansprüche 1 oder 2, wobei die pharmazeutische Zubereitung eine Dreischicht-Tablette ist, bei der zusätzlich zu den beiden getrennten Schichten eine Trennschicht vorhanden ist.

5. Pharmazeutische Festdosis-Zubereitung gemäß einem der Ansprüche 1 oder 2, wobei die pharmazeutische Zubereitung eine Gelkapsel ist, die Bisoprolol-Granulat und Perindopril-Granulat, Bisoprolol-Tabletten und Perindopril-Tabletten oder Tabletten eines der Wirkstoffe und ein Granulat des anderen Wirkstoffs enthält.

6. Pharmazeutische Festdosis-Zubereitung nach einem der Ansprüche 1 bis 5, wobei Bisoprolol in Form von Bisoprolol-Fumarat vorliegt.

7. Pharmazeutische Festdosis-Zubereitung nach einem der Ansprüche 1 oder 5, wobei Perindopril in Form von Perindopril-*tert*-Butylamin oder Perindopril-Arginin und noch bevorzugter in Form von Perindopril-Arginin vorliegt.

8. Pharmazeutische Festdosis-Zubereitung nach einem der Ansprüche 1 bis 7, wobei:
- die Dosierungen von Bisoprolol 1,05 mg und 8,50 mg, ausgedrückt als Bisoprolol-Base, oder zwischen 1,25 und 10 mg, ausgedrückt als Bisoprolol-Fumarat, betragen und
die Dosierungen von Perindopril 1,65 und 6,80 mg, ausgedrückt als Perindopril-Base, oder zwischen 2,5 bis 10 mg, ausgedrückt als Perindopril-Arginin, betragen.

9. Pharmazeutische Festdosis-Zubereitung nach einem der Ansprüche 1 bis 8, wobei die Dosierungen von Bisoprolol-Fumarat 2,5 mg, 5 mg und 10 mg betragen und die Dosierungen von Perindopril-Arginin 2,5 mg, 5 mg und 10 mg betragen.

10. Pharmazeutische Festdosis-Zubereitungen nach einem der Ansprüche 1 bis 9 zur Verwendung bei der Behandlung oder der Vorbeugung von kardiovaskulären Erkrankungen.

11. Pharmazeutische Festdosis-Zubereitungen nach Anspruch 10, **dadurch gekennzeichnet, dass** die kardiovaskulären Erkrankungen ausgewählt sind aus arterieller Hypertonie, stabiler Erkrankung der Herzkranzgefäße oder chronischer Herzinsuffizienz.

## Claims

1. Fixed pharmaceutical composition comprising bisoprolol and pharmaceutically acceptable salts thereof and perindopril and pharmaceutically acceptable salts thereof in combination with one or more pharmaceutically acceptable excipients, their hydrates and their crystalline forms, wherein the bisoprolol and the perindopril are physically separated.

2. Fixed pharmaceutical composition according to claim 1 which is a bilayer tablet in which a first layer comprises bisoprolol and a second layer comprises perindopril, a trilayer tablet in which, in addition to the two distinct layers, there is a separation layer, or a capsule containing bisoprolol granules and perindopril granules, bisoprolol tablets and perindopril tablets or tablets of one of the active ingredients and granules of the other of the active ingredients.

3. Fixed pharmaceutical composition according to either claim 1 or claim 2, wherein the pharmaceutical composition is a bilayer tablet in which a first layer comprises bisoprolol and a second layer comprises perindopril.

4. Fixed pharmaceutical composition according to either claim 1 or claim 2, wherein the pharmaceutical composition is a trilayer tablet in which, in addition to the two distinct layers, there is a separation layer.

5. Fixed pharmaceutical composition according to either claim 1 or claim 2, wherein the pharmaceutical composition is a capsule containing bisoprolol granules and perindopril granules, bisoprolol tablets and perindopril tablets or tablets of one of the active ingredients and granules of the other of the active ingredients.

6. Fixed pharmaceutical composition according to any one of claims 1 to 5, wherein the bisoprolol is in the form of bisoprolol fumarate.

7. Fixed pharmaceutical composition according to either claim 1 or claim 5, wherein the perindopril is in the form of perindopril *tert*-butylamine or perindopril arginine and more preferably in the form of perindopril arginine.

8. Fixed pharmaceutical composition according to any one of claims 1 to 7, wherein:
- the doses of bisoprolol are 1.05 mg and 8.50 mg, expressed in terms of bisoprolol base, or between 1.25 and 10 mg, expressed in terms of bisoprolol fumarate, and
- the doses of perindopril are 1.65 and 6.80 mg, expressed in terms of perindopril base, or between 2.5 and 10 mg, expressed in terms of perindopril arginine.

9. Fixed pharmaceutical composition according to any one of claims 1 to 8, wherein the doses of bisoprolol fumarate are 2.5 mg, 5 mg and 10 mg and the doses of perindopril arginine are 2.5 mg, 5 mg and 10 mg.

10. Fixed pharmaceutical compositions according to any one of claims 1 to 9 for use in the treatment or prevention of cardiovascular diseases.

11. Fixed pharmaceutical compositions according to claim 10, **characterised in that** the cardiovascular diseases are chosen from arterial hypertension, stable coronary artery disease and chronic heart failure.
